# EUROPÄISCHE PATENTSCHRIFT

(11) **EP 0 291 785 B1**
(45) Veröffentlichungstag und Bekanntmachung des Hinweises auf die Patenterteilung: **17.03.1993**
(21) Anmeldenummer: 88107306.8
(22) Anmeldetag: 06.05.1988
(51) Int. Cl.: A61B 5/02

(54) **Vorrichtung zur Vasometrie**
Device for vascular measurements
Appareil destiné à des mesures vasculaires

(30) Priorität: 21.05.1987 DE 3717045
(43) Veröffentlichungstag der Anmeldung: 23.11.1988
(73) Patentinhaber: B.V. Optische Industrie "De Oude Delft", 2600 MD Delft (NL)
(72) Erfinder: Affeldt, Karl-Heinz, Dipl.-Ing., D-1000 Berlin 19 (DE); Hantel, Ulrich, Dipl.-Ing., D-1000 Berlin 65 (DE)

(56) Entgegenhaltungen:
- FR-A- 2 296 396
- GB-A- 11 586

## Beschreibung

Die Erfindung betrifft eine Vorrichtung nach dem Oberbegriff des Anspruchs 1.

### Stand der Technik

Es ist bekannt (Prof. Völker, Herz- und Gefäßerkrankungen, Kreislaufbücherei, Band 16, Darmstadt, 1957), Durchblutungsstörungen mittels Vasographie festzustellen. Zu diesem Zweck wird zum Beispiel an einem Finger einer Hand des Probanden ein Durchlicht- oder Reflexionslichtsensor angebracht, zum Beispiel mittels eines Klettenbandes. Die mit dem Sensor durch Abtastung der Volumenpulse erhaltenen elektrischen Signale werden nach einer Verstärkung auf einem Sichtgerät als Kurvenverlauf sichtbar gemacht und gegebenenfalls mit einer Schreibvorrichtung aufgezeichnet. Durch einen Vergleich der an einem Finger der linken Hand und an einem Finger der rechten Hand erhaltenen Kurvenverläufe kann der Fachmann unter anderem Schlüsse auf den Zustand der Blutgefäße ziehen. Ein wesentlicher Nachteil der bekannten Meßmethode besteht darin, daß das Meßergebnis von der Art der Befestigung des Sensors an dem Finger sowie vom Ruhighalten des Fingers des Probanden während der Messung abhängt.

Es ist weiterhin eine Vorrichtung zum Messen des Pulses bzw. zum Erkennen von Herzrhythmusstörungen bekannt (FR-A-2 296 396), die einen in einer Grundplatte schwenkbar gelagerten Griff aufweist, in welchem ein Sensor aus einer Lampe und einem Fotowiderstand gegen eine Federkraft axial bewegbar angeordnet ist. Bei der Messung umgreift der Proband mit den Fingern seiner Hand den Griff und legt seinen Daumen auf den aus dem Griff etwas hervorstehenden Sensor.

### Aufgabe

Der Erfindung liegt die Aufgabe zugrunde, eine Vorrichtung anzugeben, die sicherstellt, daß der Proband während der Messung seine Hand möglichst locker und entspannt in einer fixierten Lage hält und daß der Sensor mit einer nur geringen mechanischen Vorspannung an dem Finger anliegt, damit das durch die Druckwelle des Blutes erzeugte Ausdehnen der Adern in dem Finger so wenig wie möglich behindert wird.

### Lösung und erzielbare Vorteile

Diese Aufgabe wird bei einer gattungsgemäßen Vorrichtung durch die kennzeichnenden Merkmale des Anspruchs 1 gelöst.

Die mit der Erfindung erzielten Vorteile bestehen insbesondere darin, daß der Proband in die Lage versetzt wird, seine Hand völlig entspannt derart auf der Vorrichtung ruhen zu lassen, daß der mit dem Sensor in Berührung stehende Finger während der Messung seine Lage unverändert beibehält und daß der Sensor durch seine federnde Lagerung nur mit einer sehr geringen Kraft auf den Finger drückt. Um eine an den Probanden optimal angepaßte Andruckkraft des Volumenpulssensors zu erhalten, ist es nach einer weiteren Ausbildung der Erfindung vorteilhaft, wenn der Volumenpulssensor derart federnd gelagert ist, daß die Federkraft einstellbar ist.

### Beschreibung eines Ausführungsbeispiels

Ein Ausführungsbeispiel der Erfindung ist in der Zeichnung anhand zweier Figuren dargestellt und wird in der Zeichnung näher beschrieben. Es zeigen
- Fig. 1: eine Schnittansicht der erfindungsgemäßen Vorrichtung und
- Fig. 2: eine Draufsicht auf die Vorrichtung nach Fig. 1.

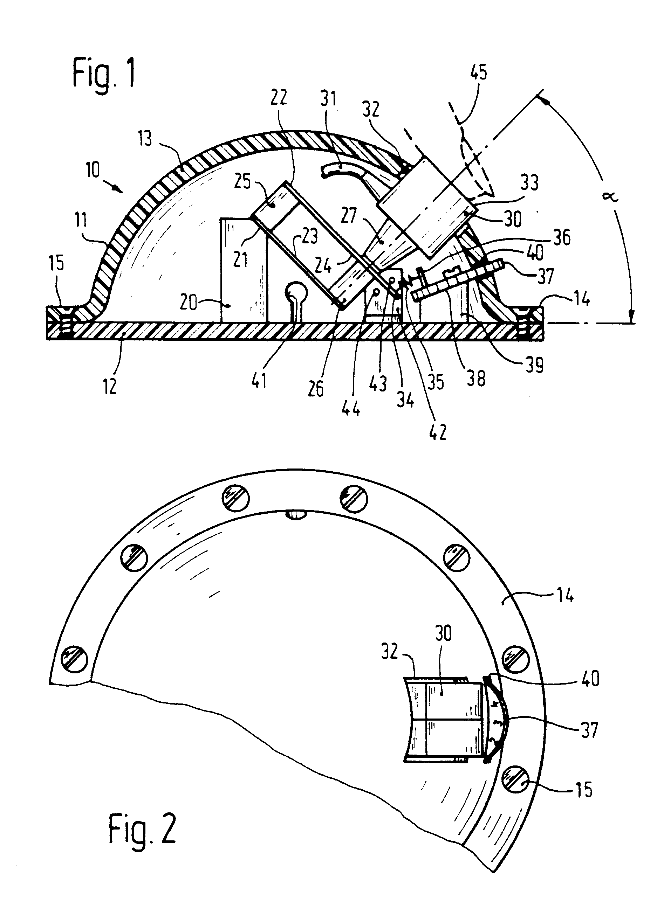

In Fig. 1 ist eine Vorrichtung 10 gezeigt, deren Gehäuse 11 aus einer kreisförmigen Grundplatte 12 und einer Kappe 13 besteht. Die Kappe hat die Form einer Kugelkalotte. Ein nach außen gerichteter Rand 14 der Kappe ist mit der Grundplatte 12 mittels über den Rand 14 in gleichmäßigen Abständen verteilt angeordneter Schrauben 15 fest verbunden. Im Innern des Gehäuses 11 ist auf der Grundplatte 12 ein Sockel 20 angeordnet, der am oberen Ende eine Abschrägung 21 aufweist, auf der ein Federteil 22 mit seinem einen Ende befestigt ist. Das Federteil besteht aus zwei länglichen Blattfedern 23 und 24, die an ihren Enden durch Abstandsstücke 25 und 26 fest miteinander verbunden sind. An dem dem Sockel 20 abgewandten Ende trägt die obere Blattfeder 24 einen Haltebügel 27 für einen etwa quaderförmigen Volumenpulssensor 30, das ist vorzugsweise ein Reflexionslichtsensor, der mit einem elektrischen Anschlußkabel 31 versehen ist. Der Volumenpulssensor ragt durch eine erste Öffnung 32 der Kappe 13 hindurch und steht mit seiner Sensoroberfläche 33 etwas aus der Oberfläche der Kappe hervor. Der Winkel α zwischen der Längsachse des Volumenpulssensors 30 und der Ebene der Grundplatte 12 beträgt vorzugsweise 40° ... 60°.

Die obere Blattfeder 24 des Federteils 22 ist etwas länger als die untere Blattfeder 23 und bildet einen Vorsprung 34, an dem ein Ende eines Federelementes 35, das ist vorzugsweise eine Zugfeder, befestigt ist. Das andere Ende der Zugfeder ist an einem Stift 36 befestigt, der sich auf einer Einstellscheibe 37 befindet. Die Einstellscheibe ist auf einer schrägen Stirnfläche 38 eines weiteren Sockels 39 drehbar gelagert und ragt mit einem Teil ihres Umfangs durch eine zweite Öffnung 40 der Kappe 13 nach außen, so daß sie mit einem Finger kontinuierlich oder stufenweise eingestellt werden kann.

Die Bewegung des Volumenpulssensors 30 begrenzt ein Anschlagwinkel 42, der auf der Grundplatte 12 befestigt ist und einen oberen Anschlagstift 43 und einen unteren Anschlagstift 44 aufweist. Im Ruhezustand liegt der Vorsprung 34 an dem oberen Anschlagstift 43 an.

Das Anschlußkabel 31 ist durch einen Einschnitt 41 in der Nähe des Randes 14 der Kappe 13 nach außen geführt und an dieser Stelle vorzugsweise mit einer dem Fachmann bekannten Zugentlastung versehen.

Die vorstehend beschriebene Vorrichtung nach den Fig. 1 und 2 hat folgende Funktion.

Der möglichst auf einer Patientenliege liegende Proband legt bei ausgestrecktem Arm eine Hand entspannt auf die seitlich neben ihm befindliche Vorrichtung 10, so daß sein Finger 45 (Fig. 1), das ist vorzugsweise sein Mittelfinger, locker auf der Sensoroberfläche 33 ruht. Die Federkraft des Federelementes 35 wird mittels der Einstellscheibe 37 so eingestellt, daß der Volumenpulssensor 30 eine sehr geringe Druckkraft auf den Finger 45 ausübt. Ist das Anschlußkabel 31 mit einem Sichtgerät und einer Stromquelle verbunden, so wandelt der Volumenpulssensor 30 die Volumenpulse in dem Finger 45 des Probanden in entsprechende elektrische Signale um, die mit dem Sichtgerät angezeigt werden.

## Patentansprüche

1. Vorrichtung zur Vasometrie im Fingerbereich der menschlichen Hand mittels eines Volumenpulssensors (30), der in einem als Auflage für die Hand bestimmten Gehäuse (11) axial federnd gelagert ist, der durch eine Öffnung (32) des Gehäuses frei hindurchtritt und mit seiner Sensoroberfläche (33) etwas über der Gehäuseoberfläche hervorsteht, **dadurch gekennzeichnet,** daß das Gehäuse (11) kalottenförmig ausgebildet ist.

2. Vorrichtung nach Anspruch 1, dadurch gekennzeichnet, daß das Gehäuse (11) aus einer ebenen Grundplatte (12) und einer damit fest verbundenen kugelförmigen Kappe (13) besteht.

3. Vorrichtung nach Anspruch 1 oder 2, dadurch gekennzeichnet, daß der Winkel (α) zwischen der Längsachse des Volumenpulssensors (30) und der Ebene der Grundplatte (12) 40° ... 60° beträgt.

4. Vorrichtung nach Anspruch 1 oder 3, dadurch gekennzeichnet, daß der Volumenpulssensor (30) ein Reflexionslichtsensor ist.

5. Vorrichtung nach Anspruch 1, 3 oder 4, dadurch gekennzeichnet, daß der Volumenpulssensor (30) an einem Ende eines Federteils (22) befestigt ist und daß das andere Ende des Federteils mit der Grundplatte (12) fest verbunden ist.

6. Vorrichtung nach Anspruch 5, dadurch gekennzeichnet, daß das Federteil (22) aus zwei länglichen Blattfedern (23, 24) besteht, die an ihren Enden durch Abstandsstücke (25, 26) fest miteinander verbunden sind.

7. Vorrichtung nach Anspruch 5 oder 6, dadurch gekennzeichnet, daß das Gehäuse (11) Anschlagmittel (42) zum Begrenzen der Bewegung des Federteils (22) enthält.

8. Vorrichtung nach einem der Ansprüche 1 bis 7, dadurch gekennzeichnet, daß der Volumenpulssensor (30) derart federnd gelagert ist, daß die Federkraft einstellbar ist.

9. Vorrichtung nach Anspruch 8, dadurch gekennzeichnet, daß das den Volumenpulssensor (30) tragende Ende des Federteils (22) über ein Federelement (35) mit einer drehbar gelagerten Einstellscheibe (37) verbunden ist, welche durch eine zweite Öffnung (40) der Kappe (13) etwas nach außen ragt.

10. Vorrichtung nach Anspruch 1 oder 2, dadurch gekennzeichnet, daß der Rand (14) der Kappe (13) des Gehäuses (11) einen Einschnitt (41) zum zugentlasteten Herausführen eines Anschlußkabels (31) des Volumenpulssensors (30) aufweist.

11. Vorrichtung nach einem der Ansprüche 1 bis 10, dadurch gekennzeichnet, daß zumindest die Kappe (13) des Gehäuses (11) aus einem wärmeisolierenden Kunststoff besteht.

## Claims

1. Apparatus for vascular measurement in the finger region of the human hand by means of a volume pulse sensor (30), which is mounted in axially resilient manner in a casing (11) intended as a support for the hand and which passes freely through an opening (32) of the casing and projects with its sensor surface (33) over the casing surface, characterized in that the casing (11) is spherical.

2. Apparatus according to claim 1, characterized in that the casing (11) comprises a planar base plate (12) and a spherical cap (13) feed thereto.

3. Apparatus according to claims 1 or 2, characterized in that the angle (α) between the longitudinal axis of the volume pulse sensor (30) and the plane of the use plate (12) is 40 to 60°.

4. Apparatus according to claims 1 or 3, characterized in that the volume pulse sensor (30) is a reflected light sensor.

5. Apparatus according to claims 1, 3 or 4, characterized in that the volume pulse sensor (30) is feed to one end of a spring part (22) and that the other end of the spring part is fixed to the base plate (12).

6. Apparatus according to claim 5, characterized in that the spring part (12) comprises two elongated leaf springs (23, 24), which are firmly interconnected at their ends by spacers (25, 26).

7. Apparatus according to claims 5 or 6, characterized in that the casing (11) contains stop means (42) for limiting the movement of the spring part (22).

8. Apparatus according to one of the claims 1 to 7, characterized in that the volume pulse sensor (30) is resiliently mounted in such a way that the spring tension is adjustable.

9. Apparatus according to claim 8, characterized in that the end of the spring part (22) carrying the volume pulse sensor (30) is connected by means of a spring element (35) to a dial (37) mounted in rotary manner and which projects somewhat to the outside through a second opening (40) in the cap (13).

10. Apparatus according to claims 1 or 2, characterized in that the edge (14) of the cap (13) of the casing (11) has a slit for the tension-relieved passing out of a connecting cable (31) of the volume pulse sensor (30).

11. Apparatus according to one of the claims 1 to 10, characterized in that at least the cap (13) of the casing (11) is made from a thermally insulating plastics material.

## Revendications

1. Dispositif pour effectuer une mesure vasculaire au niveau des doigts de la main de l'homme, à l'aide d'un capteur d'impulsions de volume (30), qui est monté de manière à être élastique axialement dans un boîtier (11) conçu pour servir d'appui pour la main, qui traverse librement une ouverture (32) du boîtier et qui fait légèrement saillie au-dessus de la surface du boîtier par sa face supérieure (33) caractérisé en ce que le boîtier (11) est réalisé sous la forme d'une calotte.

2. Dispositif selon la revendication 1, caractérisé en ce que le boîtier (11) est constitué d'une plaque de base plane (12) et d'un capot sphérique (13) raccordé rigidement à cette plaque.

3. Dispositif selon la revendication 1 ou 2, caractérisé en ce que l'angle (α) entre l'axe longitudinal du capteur d'impulsions de volume (30) et le plan de la plaque de base (12) est égal à 40°...60°.

4. Dispositif selon la revendication 1 ou 3, caractérisé en ce que le capteur d'impulsions de volume (30) est un capteur de lumière réfléchie.

5. Dispositif selon la revendication 1, 3 ou 4, caractérisé en ce que le capteur d'impulsions de volume (30) est fixé à une extrémité d'une partie élastique (22) et que l'autre extrémité de la partie élastique est raccordée rigidement à la plaque de base (12).

6. Dispositif selon la revendication 5, caractérisé en ce que la partie élastique (22) est constituée de deux ressorts en forme de lames allongées (23,24), qui sont raccordés entre eux rigidement au niveau de leurs extrémités par des entretoises (25,26).

7. Dispositif selon la revendication 5 ou 6, caractérisé en ce que le boîtier (11) contient des moyens de butée (42) pour limiter le déplacement de la partie élastique (22).

8. Dispositif selon l'une des revendications 1 à 7, caractérisé en ce que le capteur d'impulsions de volume (30) est monté élastiquement de telle sorte que la force élastique est réglable.

9. Dispositif selon la revendication 8, caractérisé en ce que l'extrémité, qui porte le capteur d'impulsions de volume (30), de la partie élastique (22) est raccordée par l'intermédiaire d'un élément de ressort (35) à un disque de réglage (37) monté rotatif, qui fait légèrement saillie vers l'extérieur à travers une seconde ouverture (40) du capot (13).

10. Dispositif selon la revendication 1 ou 2, caractérisé en ce que le bord (14) du capot (13) du boîtier (11) possède une découpe (41) permettant de faire ressortir, sans aucune contrainte de tirage, un câble raccordement (31) du capteur d'impulsions de volume (30).

11. Dispositif selon l'une des revendications 1 à 10, caractérisé en ce qu au moins le capot (13) du boîtier (11) est réalisé en une matière plastique réalisant une isolation thermique.
